# EUROPEAN PATENT APPLICATION

(11) **EP 1 746 111 A1**
(43) Date of publication of application: **24.01.2007**
(21) Application number: 05291547.7
(22) Date of filing: 19.07.2005
(51) Int. Cl.: C08F 10/00, C07F 15/04, C07C 251/38, C07D 307/52, C07D 213/38

(54) **Polymerisation catalyst system based on dioxime ligands**

(71) Applicant: TOTAL PETROCHEMICALS RESEARCH FELUY, 7181 Seneffe (Feluy) (BE); CENTRE NATIONAL DE LA RECHERCHE SCIENTIFIQUE (CNRS) Etablissement Public, 75016 Paris (FR)
(72) Inventor: Boulanger, Loise, 35690 Acigné (FR); Lavastre, Olivier, 34590 Gahard (FR); Sirol, Sabine, 1440 Wauthier-Braine (BE); Ravazi, Abbas, 7000 Mons (BE)
(74) Representative: Roufosse, Micheline C.

(57) **Abstract**

The present invention discloses active oligomerisation and polymerisation catalyst systems based on dioxime ligands.

## Description

This invention relates to the field of dioxime ligands and their use in catalyst system for the polymerisation of ethylene and alpha-olefins.

There exists a multitude of catalyst systems available for polymerising or oligomerising ethylene and alpha-olefins, but there is a growing need for finding new systems capable to tailor polymers with very specific properties. More and more post-metallocene catalyst components based on early or late transition metals from Groups 3 to 10 of the Periodic Table have recently been investigated such as for example those disclosed in Gibson and al. review (Gibson, V.C.; Spitzmesser, S.K., Chem. Rev. 2003, 103, p. 283). But there is still a need to improve either the specificities or the performances of these systems.

It is an aim of the present invention to prepare a polymerisation catalyst system based on dioxime ligands.

It is also an aim of the present invention to use dioxime ligand-based catalyst system for the homo- or co-polymerisation of ethylene and alpha-olefins.

Accordingly, the present invention uses dioxime ligands of general formula I 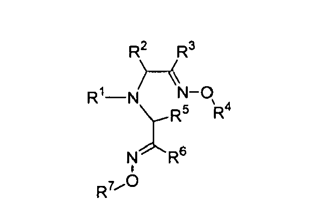 wherein R¹ R² R³ R⁴ R⁵ R⁶ and R⁷ are selected from H or alkyl having from 1 to 20 carbon atoms or aryl having from 3 to 18 carbon atoms or functional groups such as heterocycles and two neighbouring Rⁱ can be joined together to make a ring. Preferably R² and R⁵ are the same, R³ and R⁶ are the same and R⁴ and R⁷ are the same.

The dioxime ligands are prepared according to a method that comprises the steps of:
a) dissolving in a solvent a primary amine R¹-NH₂
   wherein R¹ is as described here-above,
b) suspending in the same or another solvent an oxime precursor of formula 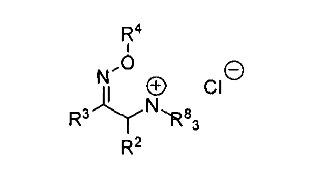 wherein R⁸ is an alkyl group and R², R³ and R⁴ are as described above;
c) reacting the primary amine with at least 2 equivalents of the oxime precursor;
d) separating the dioxime ligand from residual oxime precursor and salt by-product;
e) retrieving the dioxime ligand.

An oxime precursor TACO is described for example in Goldcamp et al. (M.J. Goldcamp, S.D. Edison, L.N. Squires, D.T. Rosa, N.K. Vowels, N.L. Coker, J.A. Krause Bauer, and M.J. Baldwin, in Inorg. Chem., 42, 717-728, 2003) or in Pavlishehuk et al. (V. V. Pavlishehuk, S.V. Kolotilov, A.W. Addison, M.J. Prushan, R.J. Butcher and L.K. Thompson, in Inorg. Chem. 38, 1759-1766, 1999).

The oxime precursor can be prepared according to the scheme

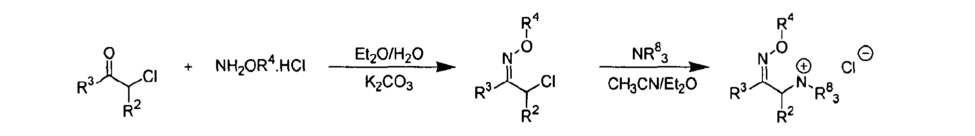

Preferably, R² and R⁴ are the same and are hydrogen, R³ is methyl and R⁸ is ethyl : this preferred precursor is called TACO.

Among the preferred embodiments according to the present invention, Rⁱ can each be independently selected from isopropyl, n-butyl, benzyl, cyclohexyl, pyridine, thiophene, furane, phenyl, mesityl.

Preferably, both the primary amine and the oxime precursor are suspended in the same solvent. The solvent is polar, preferably, it is acetonitrile.

The catalyst component is then prepared by complexing the ligand with a metallic precursor in a ratio from 1/1 to 2/1. The metallic precursor and the ligand are placed in a solvent and they are allowed to react under stirring for a period of time of from 2 to 10 hours at a temperature of from 10 to 80 °C.

The metal is selected from groups 6 to 10 of the Periodic Table. Preferably, it is Fe, Co, Cr and Ni.

The solvent may be polar or apolar, preferably it is tetrahydrofuran (THF).

An active catalyst system is then prepared by adding an activating agent having an ionising action.

Any activating agent having an ionising action known in the art may be used for activating the monooxime catalyst component. For example, it can be selected from aluminium-containing or boron-containing compounds. The aluminium-containing compounds comprise aluminoxane and/or alkyl aluminium.

The aluminoxanes are preferred and may comprise oligomeric linear and/or cyclic alkyl aluminoxanes represented by the formula: 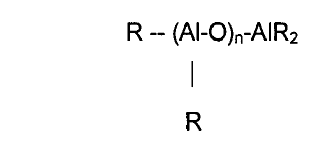 for oligomeric, linear aluminoxanes and 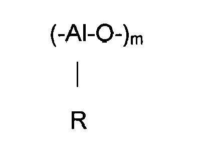 for oligomeric, cyclic aluminoxane,
wherein n is 1-40, preferably 10-20, m is 3-40, preferably 3-20 and R is a C₁-C₈ alkyl group and preferably methyl.

Suitable boron-containing activating agents that can be used comprise a triphenylcarbenium boronate such as tetrakis-pentafluorophenyl-borato-triphenylcarbenium as described in EP-A-0427696, or those of the general formula [L'-H] + [B Ar₁ Ar₂ X₃ X₄]- as described in EP-A-0277004 (page 6, line 30 to page 7, line 7).

The preferred activating agent is aluminoxane. The amount of aluminoxane necessary to activate the catalyst component is selected to have a Al/M ratio of from 100 to 3000, preferably it is about 1000.

The catalyst system can also be supported. The support if present can be a porous mineral oxide, advantageously selected from silica, alumina and mixtures thereof. Preferably it is silica.

The present invention also discloses a method for oligomerising and for homo- or co-polymerising ethylene and alpha-olefins that comprises the steps of:
a) injecting the active catalyst system into the reactor;
b) injecting the monomer and optional comonomer into the reactor;
c) maintaining under polymerising conditions;
d) retrieving the oligomers and polymers.

The polymerisation and oligomerisation methods are not particularly limited and can be carried out at a temperature of from 20 to 80 °C and under a pressure of from 5 to 50 bars.

The preferred monomers and comonomers are selected from ethylene, propylene and hexene.

### Examples.

### Synthesis of the ligand.

### a) Synthesis of the oxime precursor, TACO.

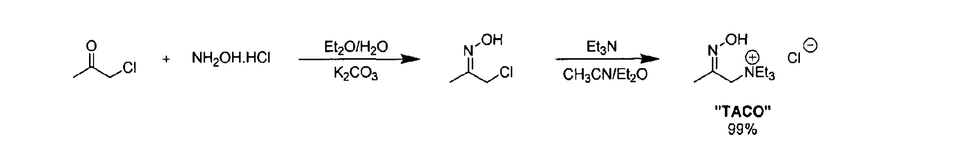

In a 250 mL flask, 3.82 g (55 mmol, 1.1 equ) of hydroxylamine hydrochloride were dissolved in 20 mL of water. A solution of 4.15 mL (50 mmol, 1 equ) of chloroacetone in 50 mL of ether was added to the flask. 3.8 g (27.5 mmol, 0.5 equ) of potassium carbonate were slowly added little by little, under stirring, at a temperature of 0 °C. The biphasic mixture was brought back to room temperature (about 25 °C) and was stirred for a period of time of 2 hours.

The two phases were then separated and the aqueous phase was extracted with 15 mL of ether. The two ether phases were combined and 7.31 g (52 mmol, 1.04 equ) of triethylamine diluted in 15 mL of acetonitrile were added drop-wise, under stirring. It was kept under stirring for a period of time of 30 minutes and gave a white precipitate that was filtered out and washed with 30 mL of cold acetonitrile. After drying under vacuum, 10.31 g of solid (TACO) were obtained with a yield of 99 %. The structure of TACO was confirmed by ¹H NMR analysis.

### b) Synthesis of dioxime ligands.

A primary amine is used to prepare ligands according to the following general scheme:

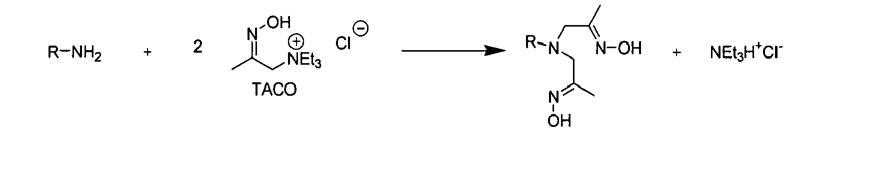

1.5 mmol (1 equ) of the primary amine was dissolved in 20 mL of acetonitrile and 689 mg of TACO (3.3 mmol, 2.2 equ) were added. The mixture was heated at a temperature of 80 °C for a period of time of 3h30. The solvent was vaporised and the residue was mixed with ethyl acetate. The mixture was then filtered to remove residual TACO and triethylamine salt and the filtrate was vaporised under vacuum.

The reaction conditions and resulting ligands are displayed in Table I.

**TABLE I.**

| **Amine** | **Ligand** | **TACO** | **Yield** |
|---|---|---|---|
| 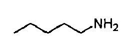 | 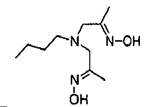 | 690 mg 3.3 mmol 2.2 éq. | >99 % |
| 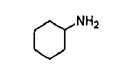 | 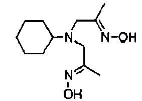 | | 97 % |
| 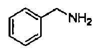 | 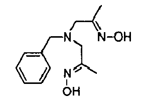 | | 98 % |
| 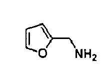 | 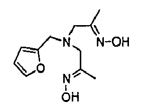 | | 98 % |
| 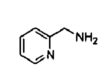 | 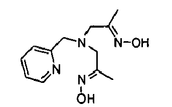 | | 63% |

### c) Polymerisation of ethylene.

Preparation of active catalyst system.

Three ligands were complexed with several metallic precursors.
Ligand L1 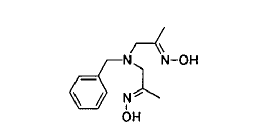
Ligand L2 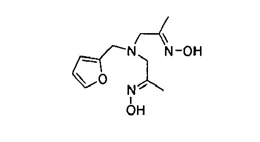
Ligand L3

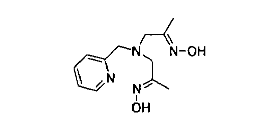

In a glovebox, a solution of 25 µmol of ligand in 6 mL of tetrahydrofuran (THF) was added to a Schlenk, followed by a solution of 25 µmol of metallic precursor in 6 mL of THF. The complexation reaction was carried out for a period of time of 4 h under stirring. THF was then removed under vacuum for a period of time of 3 h.

The catalyst component was then activated with 1000 equivalents of methylaluminoxane (MAO). 4 mL of a 30 % solution of MAO in toluene (730 equ) were added to the untreated complexation product and the mixture was kept under stirring for 5 to 10 minutes. In the reactor under inert atmosphere 50 mL of toluene were added followed by the addition of a scavenger solution prepared from 1.5 mL of a 30 % solution of MAO in toluene (270 equ) and 3.5 mL of toluene, followed by the addition of the activated complex diluted in 1 mL of toluene. The temperature was raised to 35 °C and the polymerisation of ethylene was carried out at a temperature of 35 °C and under an ethylene pressure of 15 bar, for a period of time of about 2 h.

Oligomers and polymers of ethylene were recovered after degassing. The polymers were washed with a 5 % MeOH/HCl, then with MeOH and finally with acetone. They were then dried under vacuum overnight.

The results are summarised in Table II

**TABLE II.**

| **Catalyst** | **Colour before activation** | **Colour after activation** | **Mass PE(g)** | **Polym. time (min)** | **C2 consumption (kg/h.mol)^{(a)}** | **Activity (kg PE/h.mol)** | **T_{f}^{(d)} (°C)** |
|---|---|---|---|---|---|---|---|
| Ni(DME)Br₂ + **L1** | **green** | **orange** | 1.1 | 152 | 454 | 17.4*^{(b)}* | 112 and 112 and |
| Ni(DME)Br₂ + **L2** | **green** | **orange** | 0.11 | 143 | 610 | 1.8*^{(b)}* | 112 |
| Ni(DME)Br₂ + **L3** | **green** | **orange** | 0.17 | 130 | 391 | 3.1*^{(b)}* | 111 |
| CoCl₂ + **L3** | **Pink (violet in THF)** | **salmon** | 0.21 | 99 | / | 8.4*^{(c)}* | 138 |
| FeCl₃ + **L1** | **orange** | **plum** | 0.84 | 145 | / | 33.6*^{(c)}* | 140 |
| CrCl₃(THF)₃ + **L1** | **green** | **yellowish** | 1.41 | 126 | / | 56.4*^{(c)}* | 139 |

| | | | | | | | |
|---|---|---|---|---|---|---|---|
| (a) measured after 1 h (b) continuous consumption (c)calculated on the basis of the total amount of PE obtained after 2 hours but adjusted to 1 h as the system did not show any significant evolution. (d) Melting point measured by Differential Scanning Calorimetry (DSC) method. | | | | | | | |

The complexes based on nickel produced both oligomers and polymers. The complexes based on Fe and Cr produced no significant amounts of oligomers and an improved activity with ligand L1.

## Claims

1. A dioxime ligand of general formula I 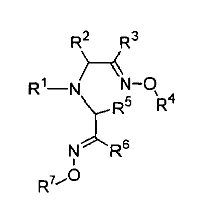 wherein R¹, R², R³, R⁴, R⁵, R⁶ and R⁷ are selected from H or an alkyl having from 1 to 20 carbon atoms, or an aryl having from 3 to 18 carbon atoms or functional groups such as heterocycles and two neighbouring R¹ can be joined together to make a ring.

2. The dioxime ligand of claim 1 wherein R² and R⁵ are the same, R³ and R⁶ are the same and R⁴and R⁷ are the same.

3. The dioxime ligand of claim 1 or claim 2 wherein R¹, R², R³, R⁴, R⁵, R⁶ and R⁷ are selected from hydrogen, methyl, isopropyl, n-butyl, benzyl, cyclohexyl, pyridine, thiophene, furane, phenyl, mesityl.

4. A method for preparing a dioxime ligand that comprises comprises the steps of:
a) dissolving in a solvent a primary amine R¹-NH₂ wherein R¹ is as described here-above,
b) suspending in the same or another solvent an oxime precursor of formula 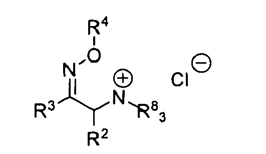
c) reacting the primary amine with at least 2 equivalents of the oxime precursor;
d) separating the dioxime ligand from residual oxime precursor and salt by-product;
e) retrieving the dioxime ligand.

5. A method for preparing a catalyst component by complexation reaction of a metallic precursor and the dioxime ligand of any one of claims 1 to 3, wherein the metal is selected from Groups 6 to 10 of the periodic Table.

6. The method of claim 5 wherein the metal in the metallic precursor is Ni, Co, Fe or Cr.

7. A catalyst component obtainable by the method of claim 5 or claim 6.

8. An active catalyst system comprising the catalyst component of claim 7 and an activating agent having an ionising action.

9. The active catalyst system of claim 8 wherein the activating agent is aluminoxane.

10. A method for homo- or co-polymerising ethylene or alpha olefins that comprises the steps of
a) injecting the active catalyst system of claim 8 or claim 9 into the reactor;
b) injecting the monomer and optional comonomer into the reactor;
c) maintaining under polymerising conditions;
d) retrieving the polymers.

11. The method of claim 10 wherein the alpha-olefin is propylene or 1-hexene.
